(19) Europäisches Patentamt
European Patent Office
Office européen des brevets

(11) EP 1 712 906 A1

(12) EUROPEAN PATENT APPLICATION
published in accordance with Art. 158(3) EPC

(43) Date of publication:
**18.10.2006 Bulletin 2006/42**

(21) Application number: **04807587.3**

(22) Date of filing: **22.12.2004**

(51) Int Cl.:
***G01N 30/88*** *(2006.01)*

(86) International application number:
**PCT/JP2004/019230**

(87) International publication number:
**WO 2005/071397 (04.08.2005 Gazette 2005/31)**

(84) Designated Contracting States:
**AT BE BG CH CY CZ DE DK EE ES FI FR GB GR HU IE IS IT LI LT LU MC NL PL PT RO SE SI SK TR**

(30) Priority: **22.12.2003 JP 2003425664**

(71) Applicant: **SHOWA DENKO KABUSHIKI KAISHA
Tokyo 105-8518 (JP)**

(72) Inventors:
- **FUJIMOTO, Etsuo; c/o Corporate R & D Center, Kawasaki-shi, Kanagawa; 2100867 (JP)**
- **SHINODA, Akiko; c/o SHOWA DENKO K.K., 5-1, 2100867 (JP)**
- **MORIKAWA, Kohei; c/o SHOWA DENKO K.K., 5-1, 2100867 (JP)**

(74) Representative: **Strehl Schübel-Hopf & Partner
Maximilianstrasse 54
80538 München (DE)**

# (54) GRANULAR POLYSACCHARIDE POLYMER HAVING PHTHALOCYANINE SKELETON BONDED THERETO

(57) This invention provides a granular polysaccharide polymer having phthalocyanine bonded thereto which is a crosslinked polymer comprising a phthalocyanine skeleton covalently bonded to a crosslinked granular porous chitosan. When this crosslinked polymer is used as an adsorbent, a polycyclic organic material present as a mixture in a solution can be selectively adsorbed, desorbed, or separated. The granular polysaccharide polymer having phthalocyanine bonded thereto is excellent not only in the ability to adsorb polycyclic organic materials, but in the ability to desorb the adsorbed polycyclic organic materials. Accordingly, the crosslinked polymer is particularly useful for selective adsorption, desorption/concentration, or separation of polycyclic organic materials, e.g., mutagens, present in a very small amount, for example, in environments, foods, table luxuries, biological samples and can be widely utilized for the qualitative determination, quantitative determination, or removal of mutagens.

EP 1 712 906 A1

## Description

### TECHNICAL FIELD

The present invention relates to a granular polysaccharide polymer having a phthalocyanine skeleton bonded thereto that is useful for the removal and analysis of polycyclic organic materials present in very small amounts as a mixture, for example, in environments and foods. Further, the present invention relates to a method for selectively adsorbing, desorbing, or separating polycyclic organic materials, particularly mutagens, present as a mixture in a solution, by using a granular polysaccharide polymer having a phthalocyanine skeleton bonded thereto as an adsorbent, and a tool for use in said method.

### BACKGROUND ART

In recent years, due to increased death rate from cancer, attention has been directed to mutagens present in a very small amount as a mixture, for example, in environments and foods. Accordingly, the development of a technique for removing these materials and a technique for analyzing these materials is a very important goal to be attained. Conventional methods useful for selectively adsorbing and removing such mutagens and for desorbing and concentrating such mutagens include, for example, methods described in patent document 1 and patent document 2. In these methods, materials produced by chemically bonding a phthalocyanine skeleton to naturally occurring polymers or organic materials, for example, polysaccharides such as Sepharose, cellulose such as paper or cotton, and polyamides such as wool, silk, or nylon are used as adsorbents.

Patent document 3 and non-patent document 1 disclose an adsorbent comprising a phthalocyanine skeleton bonded to chitosan. Chitosan used in these methods is in a powder, flake or fiber form and is not porous. That is, due to its small surface area, the adsorption speed is disadvantageously low.

Patent document 4 and patent document 5 disclose systems comprising a phthalocyanine skeleton supported on an ion exchange resin. It is, however, likely that these systems are unfavorably influenced by pH.

Further, patent document 6 and patent document 7 disclose systems comprising a phthalocyanine skeleton supported on an inorganic carrier such as silica and glass beads. These systems, however, are disadvantageous in that, since the carrier is an inorganic base material, the phthalocyanine holding power and organic compound adsorbing power are low.

Hereafter, meeting a demand for coping with an increase in types of chemical substances to be removed and detection and analysis of chemical substances present in a lower concentration is expectedly required. To this end, diversifying treatment conditions and improving treatment speed have been desired.

Patent document 1: Japanese Patent Publication No. 13481/1986
Patent document 2: Japanese Patent Publication No. 1540/1987
Patent document 3: Japanese Patent Laid-Open No. 72501/1991
Patent document 4: Japanese Patent Publication No. 7817/1989
Patent document 5: Japanese Patent Publication No. 698/1992
Patent document 6: Japanese Patent Publication No. 15036/1994
Patent document 7: Japanese Patent Laid-Open No. 148860/1992
Non-patent document 1: Water Research, 29(1) pp 101 to 105 (1995)

### DISCLOSURE OF THE INVENTION

### PROBLEMS TO BE SOLVED BY THE INVENTION

An object of the present invention is to provide a granular polysaccharide polymer having a phthalocyanine skeleton bonded thereto and a process for producing the same. Another object of the present invention is to provide a separating agent using the crosslinked granular polysaccharide polymer according to the present invention. The separating agent can rapidly adsorb polycyclic organic materials, is less likely to be influenced by pH and the like, and less likely to incur a loss of the supported phthalocyanine skeleton. A further object of the present invention is to provide use of the separating agent in concentration, separation, and purification of polycyclic organic materials. More specifically, the present invention proposes the use of the separating agent as pre-treating agents for analysis, packing materials for columns in liquid chromatography, and adsorbents.

MEANS FOR SOLVING PROBLEMS

The present inventors have made extensive and intensive studies with a view to solving the above problems and, as a result, have found that a system comprising a phthalocyanine skeleton bonded to a specific granular polysaccharide polymer as a carrier can provide adsorbing/separating agents having a high level of adsorbing/separating capability. This has led to the completion of the present invention.
The present invention will be summarized below.

[1] A granular polysaccharide polymer comprising a phthalocyanine skeleton bonded to a granular porous polysaccharide polymer.
[2] The granular polysaccharide polymer having a phthalocyanine skeleton bonded thereto according to the above item [1] wherein the particle diameter of the granular polysaccharide polymer is 1 μm to 2 mm.
[3] The granular polysaccharide polymer having a phthalocyanine skeleton bonded thereto according to the above item [1] or [2] wherein the granular polysaccharide polymer is crosslinked.
[4] The granular polysaccharide polymer having a phthalocyanine skeleton bonded thereto according to any of the above items [1] to [3] wherein said granular polysaccharide polymer is a granular porous chitosan or granular porous chitin.
[5] The granular polysaccharide polymer having a phthalocyanine skeleton bonded thereto according to any of the above items [1] to [4] wherein the granular polysaccharide polymer has a BET surface area of not less than 10 $m^2/g$.
[6] The granular polysaccharide polymer having a phthalocyanine skeleton bonded thereto according to any of the above items [1] to [5] wherein the amount of the bound phthalocyanine skeleton is 5 μmol to 1 mmol per g of the granular polysaccharide polymer.
[7] The granular polysaccharide polymer having a phthalocyanine skeleton bonded thereto according to any of the above items [1] to [6] wherein the phthalocyanine skeleton and the granular polysaccharide polymer are bonded to each other through a covalent bond.
[8] The granular polysaccharide polymer having a phthalocyanine skeleton bonded thereto according to the above item [7] wherein the phthalocyanine skeleton and the granular polysaccharide polymer are bonded to each other through a covalent bond utilizing a hydroxyl group and/or an amino group in the granular polysaccharide polymer.
[9] The granular polysaccharide polymer having a phthalocyanine skeleton bonded thereto according to the above item [8] wherein the phthalocyanine skeleton and the granular polysaccharide polymer are bonded to each other through a covalent bond utilizing a reaction between a hydroxyl group and/or an amino group in the granular polysaccharide polymer and a group reactive with the hydroxyl group and/or the amino group in a phthalocyanine reactive dye containing the reactive group.
[10] The granular polysaccharide polymer having a phthalocyanine skeleton bonded thereto according to the above item [9] wherein the reactive group in the phthalocyanine reactive dye is at least one reactive group selected from dihalogenotriazines, monohalogenotriazines, trihalogenopyrimidines, sulfatoethylsulfones, dihalogenoquinoxalines, dihalogenopyridazinones, dihalophthalazines, sulfatoethylsulfone amides, mono- or dihalogenopyrimidines, acrylamide, vinylsulfone, dihalogenobenzothiazoles, methylolamine, and acid chlorides.
[11] The granular polysaccharide polymer having a phthalocyanine skeleton bonded thereto according to the above item [10] wherein said reactive group is in a phthalocyanine reactive dye bonded to a phthalocyanine nucleus through a divalent group.
[12] A process for producing a granular polysaccharide polymer having a phthalocyanine skeleton bonded thereto according to any of the above items [1] to [11] wherein the hydroxyl group and/or the amino group in the granular polysaccharide polymer are reacted with the reactive group in the phthalocyanine reactive dye.
[13] The process for producing a granular polysaccharide polymer having a phthalocyanine skeleton bonded thereto according to the above item [12] wherein the reactive group in the phthalocyanine reactive dye is at least one reactive group selected from dihalogenotriazines, monohalogenotriazines, trihalogenopyrimidines, sulfatoethylsulfones, dihalogenoquinoxalines, dihalogenopyridazinones, dihalophthalazines, sulfatoethylsulfone amides, mono- or dihalogenopyrimidines, acrylamide, vinylsulfone, dihalogenobenzothiazoles, methylolamine, and acid chlorides.
[14] A granular polysaccharide polymer having a phthalocyanine skeleton bonded thereto, for use in concentration, purification or separation of a polycyclic organic material wherein a granular polysaccharide polymer having a phthalocyanine skeleton bonded thereto according to any of the above items [1] to [11] is used.
[15] A compound-separating tool characterized by comprising a granular polysaccharide polymer having a phthalocyanine skeleton bonded thereto according to any of the above items [1] to [11].
[16] The compound-separating tool according to the above item [15] which is a column, a cartridge, a disk, a filter, a plate, or a capillary.
[17] The compound-separating tool according to the above item [15] or [16] wherein said compound-separating tool is used in concentration, purification or separation of a polycyclic organic material.

[18] The compound-separating tool according to the above item [17] wherein said polycyclic organic material is one or at least two compounds selected from aromatic or heterocyclic compounds having two or more rings.

[19] A method for concentrating a polycyclic organic material, characterized by comprising adsorbing a polycyclic organic material on a granular polysaccharide polymer having a phthalocyanine skeleton bonded thereto according to any of the above items [1] to [11] and then desorbing the adsorbed polycyclic organic material.

[20] The method for concentrating a polycyclic organic material according to the above item [19] wherein, after the adsorption of the polycyclic organic material on the granular polysaccharide polymer having a phthalocyanine skeleton bonded thereto according to any of the above items [1] to [11] in a polycyclic organic material-containing gas or liquid, the adsorbed polycyclic organic material is desorbed by elution with a solvent.

[21] The method for concentrating a polycyclic organic material according to the above item [19] or [20] wherein said polycyclic organic material is one or at least two compounds selected from aromatic or heterocyclic compounds having two or more rings.

[22] A method for separating a polycyclic organic material,
characterized by comprising adsorbing a polycyclic organic material on a granular polysaccharide polymer having a phthalocyanine skeleton bonded thereto according to any of the above items [1] to [11] and then desorbing the adsorbed polycyclic organic material.

[23] The method for separating a polycyclic organic material according to the above item [22] wherein, after the adsorption of the polycyclic organic material on the granular polysaccharide polymer having a phthalocyanine skeleton bonded thereto according to any of the above items [1] to [11] in a polycyclic organic material-containing gas or liquid, the adsorbed polycyclic organic material is desorbed by elution with a solvent.

[24] The method for separating a polycyclic organic material according to the above item [22] or [23] wherein said polycyclic organic material is one or at least two compounds selected from aromatic or heterocyclic compounds having two or more rings.

EFFECT OF THE INVENTION

A granular polysaccharide polymer having phthalocyanine bonded thereto according to the present invention can provide a porous granular polysaccharide polymer possessing desired properties including the degree of crosslinking, and porosity by properly adjusting, for example, the type of polysaccharide, composition, and crosslinking conditions. The phthalocyanine-bonded granular polysaccharide polymer according to the present invention can provide a separating agent less likely to be influenced by pH and the like, has excellent mechanical strength, and a high level of ability to adsorb various materials. The granular polysaccharide polymer having phthalocyanine bonded thereto provided by the present invention is particularly excellent not only in the ability to adsorb polycyclic organic materials, but in the ability to desorb the adsorbed polycyclic organic material. Further, the present invention can provide a separating agent less likely to cause the elimination of the supported phthalocyanine skeleton.

BEST MODE FOR CARRING OUT THE INVENTION

The "granular polysaccharide polymer" used in the present invention can be prepared by using polysaccharides or derivatives thereof having affinity for organic materials, for example, cellulose, agarose, dextrin, chitosan, chitin or the like as a starting material. Particularly preferred are polysaccharides or derivatives thereof which are porous, have a large phthalocyanine bonding amount, and can provide a gel material having excellent moldability. From this viewpoint, granular porous chitosan or granular porous regenerated chitin derived from chitin or chitosan as a starting material is desired.

Chitin is a component constituting the shell of crustaceans such as prawns or shrimps or lobsters and crabs and has a chemical structure of a polysaccharide formed upon β-(1-4) condensation comprising N-acetyl-D-glucosamine as fundamental units. On the other hand, chitosan is a substance prepared by heating chitin together with an alkaline solution in a certain concentration range, for example, an aqueous sodium hydroxide solution, to hydrolyze chitin. Chitosan has a chemical structure of a polysaccharide formed upon β-(1-4) condensation comprising D-glucosamine as fundamental units.

Chitin is very highly crystalline and has a strong N-acetylamino group bond and as such is poor in processability and moldability. Unlike chitin, chitosan forms a salt in a dilute solution of an acid such as acetic acid, hydrochloric acid or phosphoric acid and is dissolved into the solution. Upon contact of the aqueous solution with an alkaline solution, chitosan is again coagulated and precipitated. A simply coagulated and precipitated product is in a flaky or powdery amorphous form and is not porous and, thus, has a very small surface area. Up to now, bead chitins and chitosans having a phthalocyanine skeleton bonded thereto have not been known. Accordingly, the present inventors have aimed at a process for producing a porous granular chitosan, which is porous and has uniform granular size, by dropping a highly concentrated solution of a low molecular chitosan into a basic solution for coagulation.

As the granular polysaccharide polymer in the present invention, for example, porous granular chitosan as obtained through the above process is preferably used. The chitosan prepared by the above process is in a substantially spherical form and has a particle diameter in the range of 1 μm to 2 mm. When this chitosan is molded into beads, the particle diameter can be selected from a wide particle diameter range mentioned above and, thus, the bead chitosan can meet various application requirements. In actual use, a particle diameter of 1 μm to 1 mm is preferred, and particles having a size exceeding 1 mm may also be crushed before use. The use of granular chitosan having a particle diameter of 3 to 500 μm is more desirable. Granular chitosan having a small and uniform particle size described below is suitable, for example, as a packing material for columns in liquid chromatography. On the other hand, in the case of granular chitosan having a large particle size is useful for the treatment of a large amount of fluid such as gas in a short time. The above particle diameter represents a number average particle diameter determined from a relative frequency of particles, that is, a number percentage, and was measured with a laser diffraction-type particle size distribution measuring device HELOS & RODOS system manufactured by Sympatec GmbH, or an acusizer model 780/DPS (a single particle optical detection method) manufactured by Particle Sizing Systems.

Chitosan coagulated and precipitated through the above process is preferably crosslinked with a crosslinking agent. If the chitosan is not crosslinked, it is likely to be again dissolved in an aqueous acidic solution and the significance of molding into granules is thereby reduced. Further, crosslinking increases the strength of particles. To prevent such redissolution, various methods for crosslinking with a crosslinking agent have been studied. Examples of crosslinking agents include: polyfunctional epoxide compounds such as ethylene glycol diglycidyl ether, diethylene glycol diglycidyl ether, and glycerol triglycidyl ether; and organic diisocyanate compounds such as 4,4'-diphenylmethane diisocyanate, 1,4-phenylene diisocyanate, 2,4-tolylene diisocyanate, naphthalene diisocyanate, 1,4-cyclohexane diisocyanate, 4, 4-dicyclohexylmethane diisocyanate, xylylene diisocyanate, isophorone diisocyanate, and hexamethylene diisocyanate. Various production processes of crosslinked chitosan molded products insoluble in the acidic aqueous solution have hitherto been studied. The chitosan-type granular polymer may be acetylated to prepare a granular crosslinked regenerated porous chitin material. These materials are already commercially easily available. Chitopearl (registered trademark) (manufactured by Fujibo Holdings, Inc.) may be included as an example of this type of crosslinked granular porous chitosan and granular porous regenerated chitin which are easily available. There are various types of Chitopearl including those having a chitin skeleton and those having a chitosan skeleton, and specific examples thereof include Chitopearl BCW, Chitopearl SH, Chitopearl HP, Chitopearl BASIC, DEAE Chitopearl, carboxylated Chitopearl, sulfonated Chitopearl, butylated Chitopearl, phenylated Chitopearl, Activated Chitopearl, and chelate Chitopearl. In the present invention, Chitopearl is not limited to any specific type. Chitopearl is advantageous in that the surface area is 20 to 200 $m^2$/g, the adsorption rate is high, and the bound phthalocyanine skeleton is less likely to be eliminated. Further, Chitopearl is stable against a change in pH (no change in shape is observed even after immersion in 3 N hydrochloric acid or 5 N sodium hydroxide at room temperature for 90 days (catalog value)).

In producing the granular phthalocyanine-bound polysaccharide polymer, a hydroxyl group and/or an amino group possessed by the granular polysaccharide polymer are reacted with a phthalocyanine type compound (preferably a phthalocyanine reactive dye) having a phthalocyanine skeleton and containing a group reactive with the hydroxyl group and/or the amino group. As a result, the phthalocyanine skeleton can be bound to the granular polysaccharide polymer through a covalent bond formed by a reaction between the hydroxyl group and/or the amino group in the granular polysaccharide polymer and the reactive group in the compound (preferably a phthalocyanine reactive dye). Among granular polysaccharide polymers having a phthalocyanine skeleton bonded thereto produced by this production process, granular polysaccharide polymers comprising a phthalocyanine skeleton and a granular polysaccharide polymer bonded to each other through a covalent bond formed by utilizing a reaction between a hydroxyl group and/or an amino group in the granular polysaccharide polymer and a reactive group in the phthalocyanine reactive dye containing a group reactive with these groups are particularly preferred.

The term "phthalocyanine skeleton" as used herein refers to a structure having the following nucleus.

[Chemical formula 1]

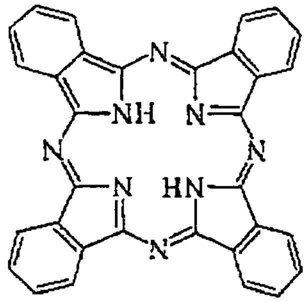

Such phthalocyanine skeletons include metal-free phthalocyanines and, further, phthalocyanines containing metals such as copper, iron, nickel, cobalt, zinc, aluminum, vanadium, manganese, and molybdenum.

Examples of compounds having a phthalocyanine skeleton and containing a group reactive with a hydroxyl or amino group (a reactive group) include phthalocyanines with a dihalogenotriazine group introduced thereinto. They may be produced by a reaction of an aminophthalocyanine compound with halogenotriazine, or by a reaction of phthalocyanine sulfonic acid chloride or phthalocyanine carboxylic acid chloride with aminotriazine. For example, a phthalocyanine with a dichloro-1,3,5-triazinyl group introduced thereinto is produced by reacting copper phthalocyanine tetrasulfone chloride as the phthalocyaninesulfonic acid chloride, which may be produced by a method described in U.K. Patent No. 515637, with 2-amino- or 2-methylamino-4,6-dichloro-1,3,5-triazine according to a method described in Japanese Patent Publication No. 5436/1959.

Regarding the reactive group-containing phthalocyanine compound preferably used in the present invention, the use of phthalocyanine reactive dyes well known in the dye industry is advantageous. The reactive dyes referred to herein are compounds defined as "dyes that contain, in the molecule thereof, an active group capable of forming a covalent bond with a fiber and can provide fast dyed products" (Makoto Okawara, Teijiro Kitao, Tsuneaki Hirashima, and Masaru Matsuoka, eds, "Shikiso Handbook (Organic Colorants)," March 20, 1986, published by Kodansha Ltd.). Examples of phthalocyanine reactive dyes which are reactive group-containing phthalocyanine compounds include dyes in which a dihalogenotriazine, monohalogenotriazine, trihalogenopyrimidine, sulfatoethylsulfone, dihalogenoquinoxaline, dihalogenopyridazinone, dihalophthalazine, sulfatoethylsulfone amide, mono- or dihalogenopyrimidine, acrylamide, vinyl sulfone, dihalogenobenzothiazole, methylolamine or other reactive group, or a reactive group having the above reactive group as a part of the structure is bonded to a phthalocyanine nucleus either directly or through a divalent group, that is, a covalent bond spacer.

The following groups may be included as specific examples of reactive groups.

Dihalogenotriazine: (dichloro-1,3,5-triazinyl)

[Chemical formula 2]

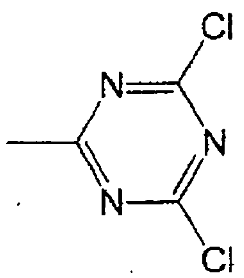

Monohalogenotriazines: (monochloro-1,3,5-triazinyl)

[Chemical formula 3]

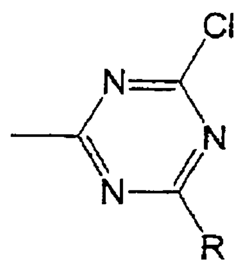

R: various substituents
Trihalogenopyrimidine: (2,4,5-trichloropyrimidinyl)

[Chemical formula 4]

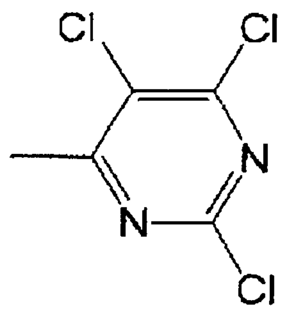

Sulfatoethylsulfone:
$-SO_2CH_2CH_2OSO_3H$ (β-sulfatoethylsulfonyl)
$-SO_2CH_2CH_2Cl$ (β-chloroethylsulfonyl)
Dihalogenoquinoxaline: (2,3-dichloroquinoxaline-6-carbonyl)

[Chemical formula 5]

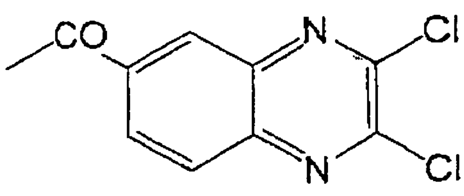

Sulfatoethylsulfone amide:
$-SO_2NHC_2H_4OSO_3H$ (β-sulfatoethylaminosulfonyl)
Mono- or dihalogenopyrimidine:
(2-methylsulfonyl-4-methyl-5-chloropyrimidinyl)

[Chemical formula 6]

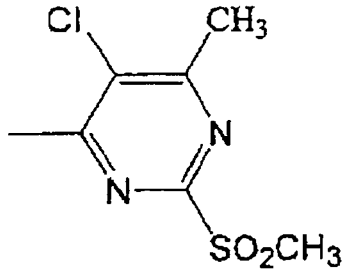

(2,4-Dichloropyrimidinyl)

[Chemical formula 7]

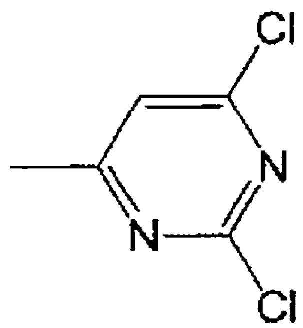

Dihalophthalazine: (1,4-dichlorophthalazine-6-carbonyl)

[Chemical formula 8]

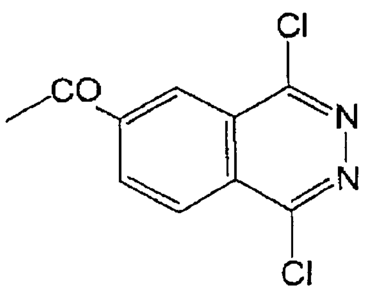

Dihalogenopyridazinone: (4,5-dichloro-2,3-dihydro-3-pyridazinone-2-propionyl)

[Chemical formula 9]

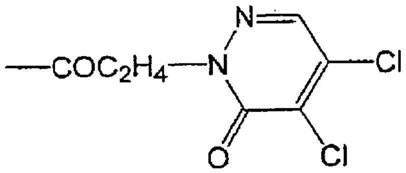

Acrylamide:
$-NHCOCH_2CH_2OSO_3H$ (β-sulfatopropionylamide)
$-NHCOCH_2CH_2Cl$ (β-chloropropionylamide)
Vinyl sulfone:
$-SO_2CH=CH_2$ (vinylsulfonyl)
Methylolamide:
$-NHCH_2OH$
Halogenobenzothiazole: (chlorobenzothiazolyl)
[Chemical formula 10]

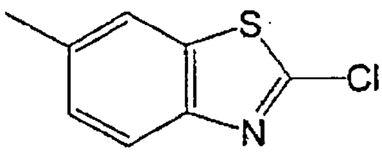

Compounds containing a reactive group such as sulfonic acid chloride or carboxylic acid chloride and having a phthalocyanine skeleton may also be referred to as compounds containing a group reactive with a hydroxyl or amino group.

Such reactive group-containing reactive dyes are described, for example, in Japanese Patent Publication No. 5436/1959, Japanese Patent Publication No. 12780/1960, Japanese Patent Publication No. 5033/1963, Japanese Patent Publication No. 17676/1964, Japanese Patent Publication No. 7782/1965, and Japanese Patent Publication No. 1027/1972.

Commercially available reactive dyes include: KAYACELON REACT TURQUOISE CN-2G, KAYACION TURQUOISE E-NA, and KAYACION TURQUOISE P-3GF manufactured by Nippon Kayaku Co., Ltd.; DRIMARENE BRILLIANT GREEN K-5BL CDG, DRIMARENE TURQUOISE K-2B CDG, and DRIMARENE TURQUOISE CL-B GRAN manufactured by Clariant Japan; Remazol Brilliant Green 6B 175% and Remazol Turquoise Blue G 133 manufactured by DyStar Japan Ltd.; and Sumifix Supra Turquoise Blue BGF (N) and Sumifix Turquoise G (N) conc manufactured by Sumitomo Chemical Co., Ltd. In the present invention, however, the reactive dyes are not limited to these only.

The reaction between the granular polysaccharide polymer and the phthalocyanine reactive dye can be carried out by a conventional method in which a fiber material is dyed with a reactive dye. Methods usable herein include dip dyeing methods and printing methods. The dip dyeing method is a method in which a material to be dyed is dipped in a reactive dye solution (a dyeing liquid) and is then dipped in a weak alkaline solution to complete the reaction. In the dyeing liquid, water is in many cases used as a main solvent. Organic solvents may be used either solely or as a water-organic solvent mixed system. A pad dyeing method may be mentioned as one of the dip dyeing methods. In the pad dyeing method, the material to be dyed is padded with a dyeing liquid, is dried, is treated with an alkali, and is heat treated to complete the reaction, or alternatively the material to be dyed is padded with an alkali-containing dyeing liquid, is dried, and is heat treated to complete the reaction. On the other hand, the printing method is a method in which dyeing is carried out using a printing paste (a colloidal solution having high viscosity) unlike the dip dyeing method in which a solvent is used as the medium. In many cases, an aqueous sodium alginate solution containing sodium bicarbonate, urea and the like is used as a printing paste in the case where a reactive dye is used. The formulation of a printing paste

varies depending upon the material to be dyed or the type of the reactive dye, and, thus, the printing paste is not limited to this composition.

In the granular polysaccharide polymer having a phthalocyanine skeleton bonded thereto according to the present invention, preferably, the phthalocyanine skeleton and the granular polysaccharide polymer are bonded to each other through a covalent bond. Since the phthalocyanine skeleton is bonded to the granular polysaccharide polymer through a covalent bond, there is no fear of causing phthalocyanine to be lost from the polymer, for example, by washing. For example, a covalent bond utilizing a hydroxyl group and/or an amino group in the granular polysaccharide polymer may be mentioned as this type of covalent bond. More preferred are granular polysaccharide polymers comprising a phthalocyanine skeleton and a granular polysaccharide polymer bonded to each other through a covalent bond formed by utilizing a reaction between a hydroxyl group and/or an amino group in the granular polysaccharide polymer and a reactive group in the phthalocyanine reactive dye containing a group reactive with these groups. The reactive group in the phthalocyanine reactive dyes is as described above.

The bonding amount of the phthalocyanine skeleton may be 5 μmol to 1 mmol per g of the granular polysaccharide polymer, preferably 10 to 500 μmol per g of the granular polysaccharide polymer, more preferably 20 to 200 μmol per g of the granular polysaccharide polymer. When the bonding amount is below the lower limit of the above-defined range, the amount of polycyclic organic materials which can be adsorbed is so small that the contemplated effect cannot be obtained. A larger bonding amount of phthalocyanine apparently effects to increase the amount of polycyclic organic materials which can be adsorbed. In fact, however, the boundable phthalocyanine amount is limited. The theoretical maximum bonding amount of phthalocyanine which can be bonded to the granular chitosan polymer can be estimated as follows. One unit of chitosan is represented with composition formula $C_6H_{11}NO_4$, and the molecular weight is 161. The maximum bonding amount is provided in the case where phthalocyanine is bonded to the amino group and the primary OH group, that is, 2 moles of phthalocyanine is bonded to one unit of chitosan. Accordingly, 12.4 mmol of phthalocyanine can be bonded to 1 g of the granular chitosan polymer. Since, however, actual granular chitosan polymer is crosslinked with a crosslinking agent, it cannot be simply said that the molar amount of phthalocyanine calculated from the molecular weight of one unit is contained in the granular chitosan polymer. Further, chitosan, which is present within the granular polymer and is not exposed on the surface of the granular polymer, is also present. In some cases, phthalocyanine cannot be bonded even to all the amino and primary OH groups present on the surface of chitosan, for example, by steric hindrance. Accordingly, the maximum bonding amount is substantially about 1 mmol.

[Chemical formula 11]

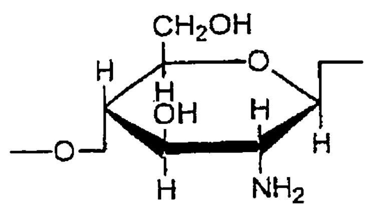

The granular polysaccharide polymer having a phthalocyanine skeleton bonded thereto is excellent in the ability to adsorb polycyclic organic materials, as well as in the ability to desorb the adsorbed polycyclic organic materials. The granular polysaccharide polymer having a phthalocyanine skeleton bonded thereto is particularly effective for polycyclic organic materials having three or more rings among polycyclic organic materials. The reason for this is probably that the arrangement of phthalocyanine bonded to within the porous crosslinked polymer is different from that in conventional polymers comprising phthalocyanine bonded in a pendant form (Japanese Patent Laid-Open No. 72501/1991) and, for example, the steric positional relationship of phthalocyanine is in such a configuration that enhances interaction with π electrons of the polycyclic compound.

Accordingly, the granular polysaccharide polymer having a phthalocyanine skeleton bonded thereto according to the present invention is suitable for use in concentration, purification, or separation of polycyclic organic materials. Specifically, a polycyclic organic material can be advantageously concentrated or separated by adsorbing the polycyclic organic material on the phthalocyanine skeleton-bonded granular polysaccharide polymer and then desorbing the adsorbed polycyclic organic material. Efficient adsorption of a polycyclic organic material and, in addition, concentration or separation of the polycyclic organic material in a short elution time without using a large amount of a solvent can be realized, for example, by adsorbing a polycyclic organic material on the phthalocyanine skeleton-bonded granular polysaccharide polymer in a polycyclic organic material-containing gas or liquid and eluting and desorbing the adsorbed polycyclic organic material with a solvent.

Specifically, the polycyclic organic material is adsorbed in a phthalocyanine skeleton-bonded granular polysaccharide polymer, for example, by adding a phthalocyanine skeleton-bonded granular polysaccharide polymer to a polycyclic organic material-containing solution, particularly aqueous solution, and then by subjecting the mixture to agitation, shaking and the like generally at 0 to 100°C, preferably 15 to 30°C. Alternatively, a method may also be adopted which comprises packing a column with a phthalocyanine skeleton-bonded granular polysaccharide polymer and passing each polycyclic organic material-containing solution through the column.

The polycyclic organic material adsorbed on the phthalocyanine skeleton-bonded granular polysaccharide polymer may also be desorbed by using a neutral, weakly alkaline or weakly acidic solution, for example, a solvent such as methanol, a methanol-hydrochloric acid solution, or an aqueous methanol-ammonia solution, and conducting agitation or shaking at a temperature at or below the boiling point of the solvent.

Thus, according to the present invention, there is also provided a method for concentrating or separating a polycyclic organic material, characterized by comprising adsorbing a polycyclic organic material (preferably one or at least two compounds selected from aromatic compounds or heterocyclic compounds having two or more rings) on a granular polysaccharide polymer having a phthalocyanine skeleton bonded thereto according to the present invention, particularly adsorbing the polycyclic organic material on the phthalocyanine skeleton-bonded granular polysaccharide polymer in a polycyclic organic material-containing gas or liquid and then desorbing the adsorbed polycyclic organic material, preferably eluting and desorbing the adsorbed polycyclic organic material with a solvent.

Examples of polycyclic organic materials which can be advantageously concentrated or separated using the phthalocyanine skeleton-bonded granular polysaccharide polymer include, for example, aromatic or heterocyclic compounds having two or more rings, and specific examples thereof include, but are not limited to, dioxins, polybiphenyl chlorides, polybiphenyl bromides, and polycyclic aromatic hydrocarbons (including PAHs and benzo(a)pyrenes), Trp-P-1 (3-amino-1,4-dimethyl-5H-pyrido[4,3-b]indole), Trp-P-2 (3-amino-1-methyl-5H-pyrido[4,3-b]indole), Glu-P-1 (2-amino-6-methyl-dipyrido[1,2-a:3',2'-d]imidazole), Glu-P-2 (2-amino-dipyrido[1,2-a:3',2'-d]imidazole), amino-α-carboline(2-amino-9H-pyrido[2,3-b]indole), aminomethyl-α-carboline(2-amino-3-methyl-9H-pyrido[2,3-b]indole), IQ (2-amino-3-methylimidazo [4,5-f]quinoline), 2-AAF (2-acetylaminofluorene), ethidium bromide, MeIQX (2-amino-3,8-dimethylimidazo-[4,5-f]quinoxaline), 9-aminoacridine, quinacrine, 8-methoxypsoralen, chlorpromazine, and Norharman (β-carboline). As a result of accumulation of a number of studies, it has been clarified that some of these polycyclic organic materials are mutagenic or carcinogenic substances to humans and animals. The "mutagenic material" or "mutagen" as used herein refers to a material that induces mutation with a higher frequency than natural mutation.

The polycyclic organic material-containing liquid or gas may be one or at least two liquids or gases selected, for example, from environmental water such as rainwater, river water, lake water, clean water, sewage, industrial waste water, and sea water; body fluids such as urine and blood or a liquid separated or extracted therefrom; extracted liquids from plant or animal tissues; incinerator waste gas, waste gas from various production facilities, environmental air such as air collected from the sky above arterial roads, or indoor air or absorption liquids prepared by passing them into absorbing liquids; foods and table luxuries such as agricultural products, marine products, animal products, and processed foods, or extracted liquids obtained by extracting them with a solvent or the like. The polycyclic organic material-containing liquid or gas, however, is not limited to these liquids and gases.

In the phthalocyanine skeleton-bonded granular polysaccharide polymer according to the present invention, when the phthalocyanine skeleton contains no metal atom, the phthalocyanine skeleton-bonded granular polysaccharide polymer is also effective in capturing heavy metals or ions thereof, for example, copper, iron, nickel, cobalt, zinc, aluminum, vanadium, manganese, and molybdenum.

<Use>

As described above, the granular polysaccharide polymer having a phthalocyanine skeleton bonded thereto according to the present invention is porous and consequently has a large surface area and thereby an increased adsorption capacity. Further, by virtue of the crosslinked structure, the strength of the granular polymer is enhanced. Moreover, by virtue of π electron interaction caused by the conveniently arranged phthalocyanine skeleton, the ability to capture polycyclic organic materials is particularly enhanced. Thus, the granular polysaccharide polymer having a phthalocyanine skeleton bonded thereto according to the present invention is excellent in the ability to adsorb, for example, materials present in very small amount as a mixture and heavy metals.

The granular polysaccharide polymer having a phthalocyanine skeleton bonded thereto, an adsorbent or separating agent, or a separating instrument, a compound-separating tool, and a reversible adsorption/desorption method using the phthalocyanine skeleton-bonded granular polysaccharide polymer are very useful in the field of research and

development or quality control of drugs and medicines and foods and drinks and environmental protection. The granular polymer having a phthalocyanine skeleton bonded thereto contains a polysaccharide as a basic skeleton of the polymer and thus is generally usable in an aqueous medium and is suitable for application to biological samples. That is, the granular polymer having a phthalocyanine skeleton bonded thereto is particularly useful for selective adsorption, desorption/concentration, and separation of polycyclic organic materials, for example, mutagens, present in very small amounts, for example, in environment, foods, table luxuries, and biological samples. In particular, they can be widely utilized for microanalysis such as qualitative or quantitative analysis of various mutagens or for the removal of the mutagens. For example, they are useful for quantitative analysis of mutagens in river water, removal of mutagens from beef extracts, quantitative determination of mutagens in urine, and quantitative determination of mutagens in foods such as agricultural products, marine products, animal products, and processed foods. Further, they can also be utilized in tools for removing mutagens contained in the smoke of smoking, and in equipments for removing polluting substances or contaminants, for example, smoking filters and air cleaning filters, in the field of environmental hygiene.

The compound-separating tool used as the adsorbent according to the present invention will be described. This is one embodiment of the present invention, and the present invention is not limited to this embodiment only. In forming a compound-separating tool using the granular polysaccharide polymer having a phthalocyanine skeleton bonded thereto according to the present invention, the granular polysaccharide polymer having a phthalocyanine skeleton bonded thereto as such may be used as a material for the compound-separating tool. Alternatively, the granular polysaccharide polymer having a phthalocyanine skeleton bonded thereto may be held on a suitable binder for use as a molded adsorbent. The term "binder" as used herein refers to an auxiliary material to be added for connecting individual phthalocyanine skeleton-bonded granular polysaccharide polymers to each other to retain a larger given shape, and the binder is preferably chemically inert. Binders include, but are not limited to, polyethylene or poly(tetrafluoroethylene) fibers commonly used in molding into a filter or disk shape.

The molded adsorbent refers to an aggregate of individual phthalocyanine skeleton-bonded granular polysaccharide polymers bonded to each other having a large given shape provided by holding individual phthalocyanine skeleton-bonded granular polysaccharide polymers on a binder. Regarding the case of the binder, the completed filter, disk or the like corresponds to the molded adsorbent. However, the outer shape of the molded adsorbent is not limited to these only.

The compound-separating tool according to the present invention can be prepared by a method wherein at least one of the above phthalocyanine skeleton-bonded granular polysaccharide polymer and its molded adsorbent is coated onto, spread onto, packed or filled into, installed in, inserted into, or hermetically sealed into a support without or together with a binder. The expression "together with a binder" means the preparation of a molded adsorbent directly in or on the surface of the support. Examples of the preparation procedure will be described. However, it should be noted that the procedure is not limited to these examples. The term "coating" refers to mainly application with a brush, or dipping into a suspension followed by pulling-up. The term "spreading" refers to mainly dispersion in gas, liquid or solid, or spraying. The term "packing" or "filling" refers to mainly packing or filling into a hollow vessel or tube as tightly as possible. The term "installing" refers to mainly placing, accumulating, sandwiching, pressure bonding, electrodeposition, or chemically bonding. The term "insertion" refers to mainly inserting or embedding. The term "hermetical sealing" refers to mainly sealing, confining, or covering.

The style of use of the compound-separating tool is not particularly limited. For example, the compound-separating tool can be used for concentration, purification, or separation of compounds, including solid phase extraction, chromatography, or pretreatment, for example, in analyses for previously carrying out concentration, removal of contaminants and the like, for example, for concentration, purification or separation of one or at least two compounds selected from polycyclic organic materials, especially aromatic compounds or heterocyclic compounds having two or more rings. Specific examples of forms of the compound-separating tool utilized in these applications include columns, cartridges, disks, filters, plates, capillary and the like for solid phase extraction or chromatography. For example, columns or capillaries for gas chromatography and plates for thin layer chromatography may be formed. Further, for example, well plates for solid phase extraction may also be formed by altering the form of the support and optionally using a binder additionally.

A specific embodiment of the compound-separating tool is one in which a syringe-type vessel (referred to as "reservoir") formed of polyethylene or the like and equipped with a pair of upper and lower filters is packed with the phthalocyanine skeleton-bonded granular polysaccharide polymer according to the present invention. Meeting the requirements that the resin reservoir is insoluble in organic solvents and the adsorbent does not flow out from the resin reservoir during concentration of a sample, suffices for the contemplated results, and the material and shape are not particularly limited. An example thereof is an assembly comprising a resin filter set in a 1 to 20 mL, preferably 3 to 6 mL, reservoir formed of, for example, polypropylene or polyethylene. The amount of the phthalocyanine skeleton-bonded granular polysaccharide polymer packed into the reservoir is generally 50 to 600 mg, preferably 200 to 500 mg, based on 6 mL of the volume of the reservoir.

A column for liquid chromatography can be prepared by packing a suitable support with the phthalocyanine

skeleton-bonded granular polysaccharide polymer according to the present invention. Meeting the requirements that the support is insoluble in an organic solvent and the phthalocyanine skeleton-bonded granular polysaccharide polymer does not leak out during concentration of a sample, suffices for contemplated results, and the material and shape are not particularly limited. A specific example of the support is a conventional one, for example, a cylindrical empty column, formed of stainless steel, polyetherether ketone or the like having an inner diameter of 1 to 20 mm and a length of 5 to 500 mm, in which an end fitting provided with a filter and a piping connection can be connected to both ends of the column. Packing of the column with the phthalocyanine skeleton-bonded granular polysaccharide polymer may be carried out by a conventional method, and the phthalocyanine skeleton-bonded granular polysaccharide polymer amount and packing conditions are adjusted so as not to yield any space between ends of the empty column. When the polysaccharide polymer is used as column packing material, the polysaccharide polymer is preferably in a granular form rather than irregular form and is particularly preferably granules having a uniform small particle diameter.

[EXAMPLES]

The present invention will be described in more detail with reference to the following examples. However, it should be noted that the present invention is not limited to these examples unless they are beyond the subject matter of the present invention.

<Measurement of specific surface area>

The specific surface area was measured with ASAP2010C manufactured by Micromeritics Instrument Corporation by the BET method.

<Measurement of metal content>

A sample was heat decomposed by sulfuric acid + nitric acid, and then the metal content was quantitatively determined by ICP-AES.

Example 1

Production of granular polysaccharide polymer A having copper phthalocyanine skeleton bonded thereto

<Step of reaction>

Ion exchanged water (500 ml) was placed in a 1 L-volume separable flask, and then placed and heated in an oil bath so that the water temperature was brought to 40°C. An anhydrous sodium sulfate (25 g) was added to and dissolved in the water. DRIMARENE TURQUISE K-2B CDG (a reactive copper phthalocyanine dye manufactured by Clariant Japan) (5 g) was then added, and the mixture was stirred at 40°C for 15 min.
Chitopearl BCW 3003 (manufactured by Fujibo Holdings, Inc.) (73.6 g; as a wet material) was added to this solution, and the mixture was stirred at 40°C for 20 min. Further, 10 g of anhydrous sodium carbonate was added to the resultant slurry. The slurry was held at 40°C for 15 min, was then heated to 80°C at a temperature rise rate of 1°C per min, was held at 80°C for 30 min, and was then allowed to cool to room temperature.

<Step of washing>

The above slurry cooled to room temperature was suction filtered to prepare a deep blue particulate material. This deep blue particulate material was placed in 500 mL of ion exchanged water, and the mixture was stirred for 5 min and was then suction filtered. This procedure was repeated five times in total. Next, the deep blue particulate material was placed in 100 mL of dimethyl sulfoxide, and the mixture was stirred for 5 min and was then suction filtered. This procedure was repeated five times in total. Subsequently, this deep blue particulate material was placed in 200 mL of methanol, was stirred for 5 min, and was then suction filtered. This procedure was repeated five times in total. Next, this deep blue particulate material was placed in 200 mL of a mixed solution composed of methanol + 30% by weight of aqueous ammonia (50% by volume of methanol vs. 1% by volume of aqueous ammonia), and the mixture was stirred for 5 min and was then suction filtered. Further, this deep blue particulate material was placed in 200 mL of methanol, and the mixture was stirred for 5 min and was then suction filtered. No coloring was observed in methanol after the filtration. Finally, this deep blue particulate material was placed in 200 mL of dimethyl ether, and the mixture was stirred and was suction filtered.

<Step of drying>

The deep blue particulate material subjected to the step of washing was dried at 70°C for 10 hr under reduced pressure to give 13.1 g of a bright blue particulate material.
The content of copper phthalocyanine in this particulate material was 82 μmol per g of the particulate material, and the BET surface area was 40 $m^2$/g.

Example 2

Production of granular polysaccharide polymer B having copper phthalocyanine skeleton bonded thereto

<Step of reaction>

A reaction with DRIMARENE TURQUISE K-2B CDG (a reactive copper phthalocyanine dye manufactured by Clariant Japan) was carried out in the same manner as in Example 1, except that Chitopearl BCW 3503 (manufactured by Fujibo Holdings, Inc.) (76.4 g; as a wet material) was used instead of Chitopearl BCW 3003 (manufactured by Fujibo Holdings, Inc.).

<Step of washing>

The step of washing was carried out in the same manner as in Example 1. In this case, no coloring was observed in the final methanol washing.

<Step of drying>

The deep blue particulate material subjected to the step of washing was dried at 70°C for 10 hr under reduced pressure to give 14.5 g of a bright blue particulate material.
The content of copper phthalocyanine in this particulate material was 55 μmol per g of the particulate material, and the BET surface area was 112 $m^2$/g.

Example 3

Production of granular polysaccharide polymer C having nickel phthalocyanine skeleton bonded thereto

<Step of reaction>

A reaction with 83.4 g (as a wet material) of Chitopearl BCW 3003 (manufactured by Fujibo Holdings, Inc.) was carried out in the same manner as in Example 1, except that DRIMARENE BRILLIANT GREEN K-5BL CDG (a reactive nickel phthalocyanine dye manufactured by Clariant Japan) was used instead of DRIMARENE TURQUISE K-2B CDG (a reactive copper phthalocyanine dye manufactured by Clariant Japan).

<Step of washing>

The resultant slurry cooled to room temperature was suction filtered to give a deep green particulate material. Subsequent washing was carried out in the same manner as in the step of washing in Example 1. Also in this case, no coloring was observed in the final methanol washing.

<Step of drying>

The deep green particulate material subjected to the step of washing was dried at 70°C for 10 hr under reduced pressure to give 12.4 g of a deep green particulate material.
The content of nickel phthalocyanine in this particulate material was 70 μmol per g of the particulate material, and the BET surface area was 10 $m^2$/g.

Example 4

Production of granular polysaccharide polymer D having nickel phthalocyanine skeleton bonded thereto

<Step of reaction>

A reaction with Chitopearl BCW 3503 (manufactured by Fujibo Holdings, Inc.) (82.1 g; as a wet material) was carried out in the same manner as in Example 1, except that DRIMARENE BRILLIANT GREEN K-5BL CDG (a reactive nickel phthalocyanine dye manufactured by Clariant Japan) was used instead of DRIMARENE TURQUISE K-2B CDG (a reactive copper phthalocyanine dye manufactured by Clariant Japan).

<Step of washing>

The resultant slurry cooled to room temperature was suction filtered to give a deep green particulate material. Thereafter, the step of washing was carried out in the same manner as in Example 1. Also in this case, no coloring was observed in the final methanol washing.

<Step of drying>

The deep green particulate material subjected to the step of washing was dried at 70°C for 10 hr under reduced pressure to give 17.4 g of a light green particulate material.
The content of nickel phthalocyanine in this particulate material was 70 μmol per g of the particulate nickel phthlocyanine and had a BET surface area of 122 $m^2$/g.

(Reference Comparative Example)

Blue chitin comprising copper phthalocyanine-trisulfonic acid bonded to chitin is available from FUNAKOSHI CO., LTD. marketed as "Blue Chitin powder." This Blue Chitin powder is a flaky form, and the BET surface area was measured with the same device as used above and was found to be as small as 0.3 $m^2$/g. An attempt was made to pack a column for liquid chromatography with the Blue Chitin powder. Due to the flaky form, however, closest packing could not be provided, resulting in a small number of theoretical plates and an increased liquid pass resistance in the column. Accordingly, the operability of the column was very poor. Further, when the Blue Chitin powder was immersed in methanol, methanol colored to blue. From this fact, it is considered that copper phthalocyanine was desorbed from the chitin. That is, this means that, for example, when the column was used as an adsorption column for mutagens, the successfully selectively adsorbed mutagens disadvantageously flow out from the column.

Example 5

Measurement of capacity ratio (capacity factor) K'

In the liquid chromatography, capacity ratio (capacity factor) K' is known as an index representing the level of retention on the packing material. This K' is regardless of column size, flow rate and the like.

$$K' = (Tr-To)/To$$

wherein K': capacity factor (capacity ratio),
Tr: retention time of target component, and
To: elution time of component not retained in packing material at all.
For Chitopearls A to D having a metal phthalocyanine bonded thereto produced in Examples 1 to 4, K' was measured. Each of the Chitopearls A to D having a metal phthalocyanine skeleton bonded thereto produced in Examples 1 to 4 was packed into a Peek (polyetherether ketone) column (inner diameter 4.6 mm, length 10 mm) by the slurry method. A liquid prepared by dissolving benzene (50 ppm), anthracene (0.5 ppm), and triphenylene (0. 5 ppm) as test compounds in an acetonitrile-water mixed liquid (acetonitrile/water = 7/3 (volume ratio)) to adjust the concentrations of these compounds to respective values within the above parentheses was analyzed by HPLC under the following conditions. The concentrations of the compounds are in ppm by weight.
Conditions for HPLC analysis:

Column; Peek (polyetherether ketone) column (inner diameter 4.6 mm, length 10 mm), single column, sample loop; 5 μL, mobile phase; acetonitrile/water = 7/3 (volume ratio), flow rate; 0.3 mL/min, column oven temp.; 40°C, and detection wavelength; 254 nm. It should be noted that, although, normally, a component, which is not retained at

all on the packing material, should be selected as To in the above equation for calculating K' , benzene was used as the index this time.

For each compound, K' was calculated based on retention times obtained by HPLC. The results are also shown in Table 1.
For comparison purposes, Chitopearl BCW 3003 not having a phthalocyanine skeleton bonded thereto and Chitopearl BCW 3503 not having a phthalocyanine skeleton bonded thereto were provided and were packed into the same Peek column as described above, and HPLC analysis was carried out in the same manner as described above. K' values for respective compounds were calculated based on the retention times thus obtained.
The results are also shown in Table 1.
[Table 1]

Table 1: Measurement of K' (capacity ratio)

| HPLC test compound | Only Chitopearl | | Example | | | |
|---|---|---|---|---|---|---|
| | Chitopearl BCW3003 | Chitopearl BCW3503 | A | B | C | D |
| Benzene | 0.00 | 0.00 | 0.00 | 0.00 | 0.00 | 0.00 |
| Anthracene | 0.03 | 0.07 | 0.22 | 0.17 | 0.30 | 0.20 |
| Triphenylene | 0.06 | 0.09 | 1.59 | 1.40 | 2.61 | 1.37 |

From Table 1, it is apparent that, for Chitopearls A to D having a metal phthalocyanine skeleton bonded thereto, K' values for anthracene and triphenylene are larger than those for Chitopearls not having a phthalocyanine skeleton bonded thereto. Thus, the porous material with a phthalocyanine skeleton bonded thereto according to the present invention, when used in solid phase extraction, can desorb the above polycyclic organic materials contained in an environment in a more rapid manner. Further, when the pore material with a phthalocyanine skeleton bonded thereto according to the present invention is used in chromatography such as HPLC, a more rapid analysis can be attained.

## Claims

1. A granular polysaccharide polymer comprising a phthalocyanine skeleton bonded to a granular porous polysaccharide polymer.

2. The granular polysaccharide polymer having a phthalocyanine skeleton bonded thereto according to claim 1 wherein the particle diameter of the granular polysaccharide polymer is 1 $\mu$m to 2 mm.

3. The granular polysaccharide polymer having a phthalocyanine skeleton bonded thereto according to claim 1 or 2 wherein the granular polysaccharide polymer is crosslinked.

4. The granular polysaccharide polymer having a phthalocyanine skeleton bonded thereto according to any of claims 1 to 3 wherein said granular polysaccharide polymer is a granular porous chitosan or granular porous chitin.

5. The granular polysaccharide polymer having a phthalocyanine skeleton bonded thereto according to any of claims 1 to 4 wherein the granular polysaccharide polymer has a BET surface area of not less than 10 $m^2/g$.

6. The granular polysaccharide polymer having a phthalocyanine skeleton bonded thereto according to any of claims 1 to 5 wherein the amount of the bound phthalocyanine skeleton is 5 $\mu$mol to 1 mmol per g of the granular polysaccharide polymer.

7. The granular polysaccharide polymer having a phthalocyanine skeleton bonded thereto according to any of claims 1 to 6 wherein the phthalocyanine skeleton and the granular polysaccharide polymer are bonded to each other through a covalent bond.

8. The granular polysaccharide polymer having a phthalocyanine skeleton bonded thereto according to claim 7 wherein the phthalocyanine skeleton and the granular polysaccharide polymer are bonded to each other through a covalent bond utilizing a hydroxyl group and/or an amino group in the granular polysaccharide polymer.

**9.** The granular polysaccharide polymer having a phthalocyanine skeleton bonded thereto according to claim 8 wherein the phthalocyanine skeleton and the granular polysaccharide polymer are bonded to each other through a covalent bond utilizing a reaction between a hydroxyl group and/or an amino group in the granular polysaccharide polymer and a group reactive with the hydroxyl group and/or the amino group in a phthalocyanine reactive dye containing the reactive group.

**10.** The granular polysaccharide polymer having a phthalocyanine skeleton bonded thereto according to claim 9 wherein said reactive group in the phthalocyanine reactive dye is at least one reactive group selected from dihalogenotriazines, monohalogenotriazines, trihalogenopyrimidines, sulfatoethylsulfones, dihalogenoquinoxalines, dihalogenopyridazinones, dihalophthalazines, sulfatoethylsulfone amides, mono- or dihalogenopyrimidines, acrylamide, vinylsulfone, dihalogenobenzothiazoles, methylolamine, and acid chlorides.

**11.** The granular polysaccharide polymer having a phthalocyanine skeleton bonded thereto according to claim 10 wherein said reactive group is in a phthalocyanine reactive dye bonded to a phthalocyanine nucleus through a divalent group.

**12.** A process for producing a granular polysaccharide polymer having a phthalocyanine skeleton bonded thereto according to any of claims 1 to 11 wherein the hydroxyl group and/or the amino group in the granular polysaccharide polymer are reacted with the reactive group in the phthalocyanine reactive dye.

**13.** The process for producing a granular polysaccharide polymer having a phthalocyanine skeleton bonded thereto according to claim 12 wherein the reactive group in the phthalocyanine reactive dye is at least one reactive group selected from dihalogenotriazines, monohalogenotriazines, trihalogenopyrimidines, sulfatoethylsulfones, dihalogenoquinoxalines, dihalogenopyridazinones, dihalophthalazines, sulfatoethylsulfone amides, mono- or dihalogenopyrimidines, acrylamide, vinylsulfone, dihalogenobenzothiazoles, methylolamine, and acid chlorides.

**14.** A granular polysaccharide polymer having a phthalocyanine skeleton bonded thereto, for use in concentration, purification or separation of a polycyclic organic material wherein a granular polysaccharide polymer having a phthalocyanine skeleton bonded thereto according to any of claims 1 to 11 is used.

**15.** A compound-separating tool **characterized by** comprising a granular polysaccharide polymer having a phthalocyanine skeleton bonded thereto according to any of claims 1 to 11.

**16.** The compound-separating tool according to claim 15 which is a column, a cartridge, a disk, a filter, a plate, or a capillary.

**17.** The compound-separating tool according to claim 15 or 16 wherein said compound-separating tool is used in concentration, purification or separation of a polycyclic organic material.

**18.** The compound-separating tool according to claim 17 wherein said polycyclic organic material is one or at least two compounds selected from aromatic or heterocyclic compounds having two or more rings.

**19.** A method for concentrating a polycyclic organic material, **characterized by** comprising adsorbing a polycyclic organic material on a granular polysaccharide polymer having a phthalocyanine skeleton bonded thereto according to any of claims 1 to 11 and then desorbing the adsorbed polycyclic organic material.

**20.** The method for concentrating a polycyclic organic material according to claim 19 wherein, after the adsorption of the polycyclic organic material on the granular polysaccharide polymer having a phthalocyanine skeleton bonded thereto according to any of claims 1 to 11 in a polycyclic organic material-containing gas or liquid, the adsorbed polycyclic organic material is desorbed by elution with a solvent.

**21.** The method for concentrating a polycyclic organic material according to claim 19 or 20 wherein said polycyclic organic material is one or at least two compounds selected from aromatic or heterocyclic compounds having two or more rings.

**22.** A method for separating a polycyclic organic material,
**characterized by** comprising adsorbing a polycyclic organic material on a granular polysaccharide polymer having a phthalocyanine skeleton bonded thereto according to any of claims 1 to 11 and then desorbing the adsorbed polycyclic organic material.

**23.** The method for separating a polycyclic organic material according to claim 22 wherein, after the adsorption of the polycyclic organic material on the granular polysaccharide polymer having a phthalocyanine skeleton bonded thereto according to any of claims 1 to 11 in a polycyclic organic material-containing gas or liquid, the adsorbed polycyclic organic material is desorbed by elution with a solvent.

**24.** The method for separating a polycyclic organic material according to claim 22 or 23 wherein said polycyclic organic material is one or at least two compounds selected from aromatic or heterocyclic compounds having two or more rings.

**INTERNATIONAL SEARCH REPORT**

International application No.
PCT/JP2004/019230

A. CLASSIFICATION OF SUBJECT MATTER
Int.Cl$^7$ G01N30/48, G01N30/88

According to International Patent Classification (IPC) or to both national classification and IPC

B. FIELDS SEARCHED

Minimum documentation searched (classification system followed by classification symbols)
Int.Cl$^7$ G01N30/48, G01N30/88

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 3-72501 A (Takeda Chemical Industries, Ltd.), 27 March, 1991 (27.03.91), (Family: none) | 1-24 |
| X | JP 5-15576 A (Takeda Chemical Industries, Ltd.), 26 January, 1993 (26.01.93), (Family: none) | 1-24 |
| X | JP 5-17478 A (Takeda Chemical Industries, Ltd.), 26 January, 1993 (26.01.93), (Family: none) | 1-24 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 May, 2005 (19.05.05) | 07 June, 2005 (07.06.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.
PCT/JP2004/019230

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-19293 A (NISSUI PHARMACEUTICAL CO., LTD.), 21 January, 2000 (21.01.00), & EP 1081717 A & WO 99/056287 A | 1-24 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 61013481 A
- JP 62001540 A
- JP 3072501 A
- JP 1007817 A
- JP 4000698 A
- JP 6015036 A
- JP 4148860 A
- GB 515637 A
- JP 54361959 B
- JP 127801960 B
- JP 50331963 B
- JP 176761964 B
- JP 77821965 B
- JP 47001027 A


### Non-patent literature cited in the description


- *Water Research,* 1995, vol. 29 (1), 101-105
- Shikiso Handbook. Kodansha Ltd, 20 March 1986